# NEUE EUROPÄISCHE PATENTSCHRIFT

(11) **EP 0 714 237 B2**
(45) Veröffentlichungstag und Bekanntmachung des Hinweises auf die Entscheidung über den Einspruch: **17.09.2003**
(45) Hinweis auf die Patenterteilung: 04.11.1998
(21) Anmeldenummer: 94924848.8
(22) Anmeldetag: 05.08.1994
(51) Int. Cl.: A01N 57/20

(54) **VERFAHREN ZUR ERTRAGSSTEIGERUNG VON HERBIZIDRESISTENTEN NUTZPFLANZEN**
METHOD OF INCREASING THE YIELD OF HERBICIDE-RESISTANT CROP PLANTS
PROCEDE POUR L'ACCROISSEMENT DU RENDEMENT DE PLANTES UTILES RESISTANT AUX HERBICIDES

(30) Priorität: 12.08.1993 DE 4327056
(43) Veröffentlichungstag der Anmeldung: 05.06.1996
(73) Patentinhaber: Bayer CropScience GmbH, 65929 Frankfurt/Main (DE)
(72) Erfinder: DONN, Günter, D-65719 Hofheim (DE)
(74) Vertreter: Tetaz, Franck Claude Edouard
(86) Internationale Anmeldenummer: EP9402598
(87) Internationale Veröffentlichungsnummer: WO95005082

(56) Entgegenhaltungen:
- EP-A- 0 242 236
- EP-A- 0 242 246
- EP-A- 0 481 407
- DE-A- 3 200 486

## Beschreibung

Die Verbindung Glufosinate (Glufosinate-ammonium, Ammonium-DL-homoalanin-4-yl(methyl)phosphinat, Schwerdtle et al., Z. Pflanzenkr. Pflanzenschutz., 1981, Sonderheft IX, Seite 431) wirkt als Inhibitor der Glutaminsynthetase (GS), da es sich um ein Strukturanalogon der Glutaminsäure handelt. Die GS spielt bei allen Pflanzen eine zentrale Rolle im Metabolismus. Sie ist für die Entgiftung von NH₃ verantwortlich ist, was zur Folge hat, daß alle Landpflanzen nach der Applikation von Glufosinate durch die unterbundene Ammoniak-Assimilation stark geschädigt bzw. abgetötet werden.

Durch die Übertragung und Expression eines Glufosinate-Acetyltransferasegens, das aus aus Bialaphos (Phosphinothricin-alanyl-alanin) produzierenden Streptomycetenstämmen isoliert wurde (EP-B1-0 242 236 und EP-B1-0 257 542), gelang es, Pflanzen herzustellen, die gegen die herbizide Wirkung von GS-Inhibitoren resistent sind. Solche transgenen, herbizidverträglichen Kulturpflanzenbestände können durch eine Behandlung mit Glufosinate im Nachauflaufverfahren effektiv unkrautfrei gehalten werden.

In Feldversuchen mit solchen transgenen Pflanzen zeigte sich unerwarteterweise, daß die mit Glufosinate behandelteten Pflanzen einen meßbar höheren Ertrag erbringen als unbehandelten Pflanzenbestände. Dieser Mehrertrag ist nicht auf die ausgezeichnete Unkrautbekämpfung durch Glufosinate und dessen vollkommene Verträglichkeit in den transgenen Kulturpflanzenbeständen zurückzuführen, sondern auf einen positiven wachstums- und ertragsbeeinflussenden Effekt der Herbizid-Behandlung.

Die Erfindung betrifft daher ein Verfahren zur Ertragssteigerung von Glutaminsynthetase-Inhibitor-resistenten Nutzpflanzen, dadurch gekenzeichnet, daß die Pflanzen mit Aufwandmengen von Glutaminsynthetase-Inhibitoren behandelt werden, die die Pflanzen nicht schädigen.

Die Erfindung betrifft insbesondere ein Verfahren, in dem Glutaminsynthetase-Inhibitoren zur ertragssteigernden Behandlung von Pflanzen, die durch die Expression eines N-Acetyltransferasegens vor der herbiziden Wirkung der Glutaminsynthetase-Inhibitoren geschützt sind, eingesetzt werden.

Die Erfindung betrifft außerdem die Verwendung eines Glutaminsynthetase-Inhibitors zur Ertragssteigerung von Nutzpflanzen, die gegen diesen Inhibitor resistent sind. Sie betrifft insbesondere die Verwendung von Glutamin-synthetase-Inhibitoren zur Ertragssteigerung von transgenen Nutzpflanzen.

Als Glutaminsynthetase-Inhibitor wird vorzugsweise die Verbindung Glufosinate oder Bialaphos verwendet (Tachibana ei al., Abstr. 5th Int. Congr. Pestic. Chem., IVa, Abstract 19; Mase, Jpn. Pestic. Inf., 1984, No 45, p. 27). Der Begriff Glufosinate umfaßt in diesem Zusammenhang sowohl das Razemat (DL-Homoalanin-4yl(methyl)phosphinsäure) als auch das biologisch aktive L-lsomer und die entsprechenden Salze. Das Herbizid kann in den handelsüblichen Formulierungen eingeseizt werden. Ein weiteres Beispiel für einen GS-Inhibitor stellt die Verbindung Phosalacin dar (Omura ei al., J. of Antibiotics, Vol. 37, 8, Seiten 939-940, 1984).

Der ertragssteigernde Effekt der Glufosinate-Behandlung tritt insbesondere dann auf, wenn die Herbizidbehandlung im 2 - 8, vorzugsweise im 3 - 6 Blattstadium der Kulturpflanzen vor der Blüte, bzw. bei mehrjährigen Pflanzen zu einem beliebigen Zeitpunkt, durchgeführt wird.

In dem erfindungsgemäßen Verfahren werden die Pflanzen mindestens einmal mit Aufwandmengen des Herbizides behandelt, wie sie auch zur Unkrautbekämpfung eingesetzt werden, z. B. mit 150 g 1000 g Glufosinate/ha.

In Abhängigkeit von der Pflanzen, deren Größe sowie den klimatischen Bedingungen kann die erforderliche Aufwandmenge jedoch variieren.

Besonders günstig kann das Verfahren bei Aufwandmengen von 350 - 700 g Glufosinate/ha eingesetzt werden. In diesem Aufwandmengenbereich ist der erzielte Effekt proportional zur Glufosinataufwandmenge, ohne auf Unterschieden im Unkrautbekämpfungserfolg zu beruhen. Bereits bei der niedrigen PTC-Aufwandmenge kann eine Unkrautkontrolle erzielt werden, die mit der Kontrolle bei höheren Aufwandmengen vergleichbar ist.

Besonders vorteilhaft ist eine mehrmalige Behandlung der Pflanzen mit geringen Dosierungen im unteren Bereich der Anwendungskonzentrationen, die im Abstand von einigen Tagen, d. h. zwischen 2 und 30 Tagen, vorzugsweise zwischen 5 und 20, besonders bevorzugt zwischen 8 und 15 Tagen durchgeführt wird. Besonders vorteilhaft ist eine Behandlung der Pflanzen mit niedrigen Dosierungen, die im Abstand von 9 bis 11 Tagen durchgeführt wird.

Das erfindungsgemäße Verfahren kann generell für die Behandlung von Pflanzen eingesetzt werden, die gegen Inhibitoren der GS resistent sind. Resistente Pflanzen können auch mit herkömmlichen Züchtungsverfahren erhalten werden. Falls die durch konventionelle Selektion erhaltenen Pflanzen ein zu den transgenen Pflanzen vergleichbares Resistenzniveau aufweisen, können sie ebenfalls nach dem erfindungsgemäßen Verfahren behandelt werden. Das Verfahren eignet sich jedoch in besonderer Weise für die Behandlung von Glufosinate-resistente Pflanzen, die durch die Übertragung eines Resistenzgens gegen das Herbizid erhalten wurden. In der EP-B1-0 242 236 und der EP-B1-0 257 542 werden Verfahren beschrieben, wie solche Pflanzen erzeugt werden können.

Der Begriff Pflanzen umfaßt in diesem Zusammenhang Nutzpflanzen aus den beiden Gruppen der Angio- und Gymnospermae. Sowohl einzelne Pflanzen als auch die Pflanzenkulturen können nach dem erfindungsgemäßen Verfahren behandelt werden.

Unter den Gymnospermae sind von besonderem Interesse die Klasse der Coniferae.

Unter den Angiospermae sind von besonderem Interesse die Pflanzen der Familien Solanaceae, Cruciferae, Compositae, Liliaceae, Vitaceae, Chenopodiaceae, Rutaceae, Bromeliaceae, Rubiaceae, Theaceae, Musaceae oder Gramineae sowie der Ordnung der Leguminosae. Bevorzugt werden Vertreter der Familien Solanaceae, Cruciferae und Gramineae behandelt.

Das Verfahren ist von besonderen Interesse für die Behandlung von Kulturpflanzen, bei denen große Ernteerträge von Bedeutung sind, wie z.B. Mais, Soja, Sommer-und Winterraps, Zuckerrübe, Luzerne, Sonnenblume, Baumwolle, Kartoffeln, Weizen, Gerste, Reis. Es kann aber auch vorteilhaft bei Tomaten und anderen Gemüsearten, wie Gurke sowie Fruchtarten wie Melone, Erdbeere, Himbeere, Kiwi, eingesetzt werden.

Ebenfalls von besonderer Bedeutung ist der Einsatz der Verfahrens in herbizidresistenten Gehölzen, wie z.B. in Plantagen und Baumschulen.

Durch die Applikation von GS-Inhibitoren, wie z. B. PTC und dessen Analoga und Derivate, auf die Jungpflanzen von Gehölzen kann deren Jugendentwicklung beschleunigt werden. Besonders Nußbäume, Ölpalmen, Obstbäume, Pappeln und anderen Anbaupflanzen, die zu den Holzgewächsen zählen, sind hier zu nennen.

Das erfindungsgemäße Verfahren ist also sowohl in der Landwirtschaft als auch im Gartenbau von Bedeutung, weil durch die Applikation des herbiziden Glutaminsynthetase-Hemmers, ohne zusätzlichen Einsatz an Dünger und Pflanzenwachstumsregulatoren, ein deutlich messbarer Mehrertrag erzielt werden kann. Der Begriff Mehrertrag bedeutet in diesem Zusammenhang, daß die Pflanze mehr Ertrag beträbt bis 50 % betragen. Herbizide mit anderen Wirkungsweisen zeigen diesen Effekt entweder nicht oder haben häufig einen negativen Effekt auf den Ertrag.

Die wachstumsfördernde Wirkung der Behandlung mit Glufosinate kann in Feld- und Gefäßversuchen gemessen werden, indem die Erträge von Pflanzenbeständen verglichen werden, die mit konventionellen Herbiziden behandelt werden oder durch nicht-chemische Verfahren unkrautfrei gehalten wurden.

Die folgenden Beispiele dienen der Erläuterung der Erfindung, ohne daß diese darauf beschränkt wäre.

### Beispiel 1

Transgene Glufosinate-tolerante Mais- bzw. Soja-Pflanzen wurden in Parzellen (10 m²) angepflanzt und im 3 - 5 Blattstadium mit unterschiedlichen Mengen Glufosinate behandelt. Die Bonitur der Unkrautbekämpfung wurde 42 Tage nach der Applikation durchgeführt. Zum Reifezeitpunkt der Kulturen wurden die Parzellen geerntet und der Samenertrag durch Auswiegen der erhaltenen Körner ermittelt.

Bei der Untersuchung der Maispflanzen wurde als Vergleichsmittel Laddock® (eine Mischung aus Atrazin und Bentazon) eingesetzt. Zur Behandlung der Sojapflanzen wurden zwei Mittel eingesetzt. Vergleichsmittel 1 enthielt eine Mischung aus 134 g Fenoxaprop-P-ethyl/ha und 425 g Fomesafen/ha; Vergleichsmittel 2 enthielt 2240 g Metolachlor/ha und 840 g Storm® (eine Mischung aus Bentazon und Acifluorfen)/ha. Die Vergleichsmittel sind aus "The Pesticide Manual", 9. Ausgabe, Brit. Crop Prot. Council, 1991 bekannt.

Die in Tabelle 2 beschriebene Behandlung mit zwei niedrigen Dosierungen von Glufosinate wurde im Abstand von 10 Tagen durchgeführt.

**Tabelle 1**

| | Glufosinate-Aufwandmengen (g Wirkstoff/ha) | | | Vergleichsmittel |
|---|---|---|---|---|
| | 150 | 450 | 650 | |
| Unkrautbekämpfungserfolg in % | 92 | 97 | 98 | 78 |
| Ertrag in % der Vergleichsmittelparzelle | 118 | 121 | 125 | 100 |

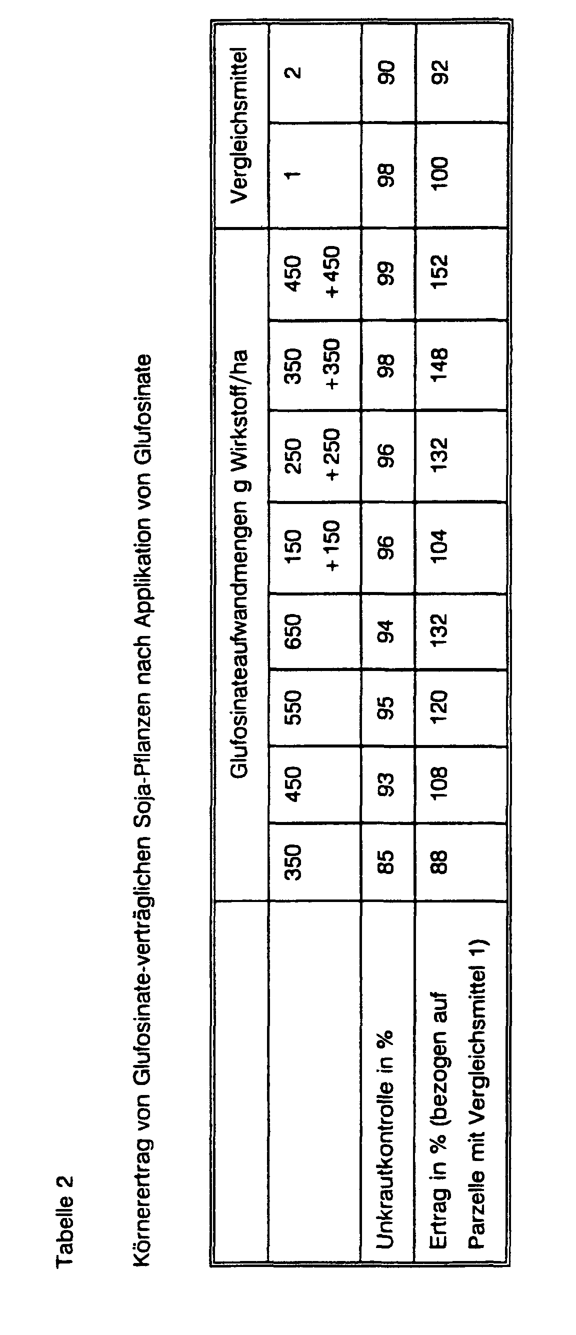

## Patentansprüche

1. Verfahren zur Ertragssteigerung von Glutaminsynthetase-lnhibitorresistenten Nutzpflanzen, **dadurch gekennzeichnet, daß** die Pflanzen mit Aufwandmengen von Glutaminsynthetase-inhibitoren behandelt werden, die die Pflanzen nicht schädigen.

2. Verfahren gemäß Anspruch 1, in dem Glufosinate und dessen Salze zur ertragssteigerenden Behandlung von Nutzpflanzen, die durch die Expression eines N-Acetyltransferasegens vor der herbiziden Wirkung der Glutaminsynthetase-Inhibitoren geschützt sind, eingesetzt werden.

3. Verfahren gemäß Anspruch 1, in dem die Nutzplanzen mindestens einmal mit Aufwandmengen des Wirkstoffs behandelt werden, wie sie auch zur Unkrautbekämpfung eingesetzt werden.

4. Verfahren gemäß Anspruch 1, in dem die Nutzpflanzen mindestens einmal mit 150-1000 g Glufosinate/ha, vorzugsweise mit 350-700 g Glufosinate/ha, behandelt werden.

5. Verwendung eines Glutaminesynthetase-Inhibitors zur Ertragssteigerung von Nutzpflanzen, die gegen diesen Inhibitor resistent sind.

## Claims

1. A method of increasing the yield of crop plants which are resistant to glutamine synthetase inhibitors, which comprises treating the plants with glutamine synthetase inhibitors at application rates which are not harmful to the plants.

2. The method as claimed in claim 1, wherein glufosinate and salts thereof are employed for the yield-increasing treatment of crop plants which are protected against the herbicidal action of glutamine synthetase inhibitors by expression of an N-acetyltransferase gene.

3. The method as claimed in claim 1, wherein the crop plants are treated at least once with application rates of the active substance as they are also employed for weed control.

4. The method as claimed in claim 1, wherein the crop plants are treated at least once using 150-1000 g of glufosinate/ha, preferably using 350-700 g of glufosinate/ha.

5. The use of a glutamine synthetase inhibitor for increasing the yield of crop plants which are resistant to this inhibitor.

## Revendications

1. Procédé visant à augmenter la récolte des plantes utiles résistantes à l'inhibiteur de glutamine synthétase, **caractérisé en ce que** l'on traite les végétaux avec l'inhibiteur de glutamine synthétase par des concentrations d'emploi qui n'endommagent pas les plantes.

2. Procédé selon la revendication 1, dans lequel on utilise le glufosinate et ses sels pour le traitement visant à augmenter la récolte en traitant les plantes utiles qui sont protégées par l'expression d'un gène de N-acétyltransférase contre l'action herbicide des inhibiteurs de glutamine synthétase.

3. Procédé selon la revendication 1, dans lequel on traite les plantes utiles au moins une fois avec des concentrations d'emploi de la substance active telles que l'on utilise pour la lutte contre les plantes nuisibles.

4. Procédé selon la revendication 1, dans lequel on traite les plantes utiles au moins une fois avec 150 à 1 000 g de glufosinate/ha, de préférence avec 350 à 700 g de glufosinate/ha.

5. Utilisation d'un inhibiteur de glutamine synthétase pour augmenter la récolte des plantes utiles résistantes à cet inhibiteur.
